# EUROPEAN PATENT APPLICATION

(11) **EP 4 667 566 A1**
(43) Date of publication of application: **24.12.2025**
(21) Application number: 24755942.0
(22) Date of filing: 26.01.2024
(51) Int. Cl.: C12N 9/78, A61K 38/50, A61K 47/60, A61P 35/00

(54) **ARGININE DEIMINASE MUTANT, COVALENT DIMER AND CONJUGATE AND USE THEREOF**

(30) Priority: 15.02.2023 CN 202310115527
(71) Applicant: Peg-Bio Biopharm Co., Ltd. (Chongqing), Chongqing 400714 (CN)
(72) Inventor: FAN, Kai, Chongqing 400714 (CN); HE, Yunfeng, Chongqing 400714 (CN); LIU, Riyong, Chongqing 400714 (CN); ZHANG, Qi, Chongqing 400714 (CN); TANG, Guanghui, Chongqing 400714 (CN); NIE, Tangwei, Chongqing 400714 (CN); DING, Qiong, Chongqing 400714 (CN); WEN, Haiyan, Chongqing 400714 (CN); BAI, Yanmin, Chongqing 400714 (CN); CHEN, Qing, Chongqing 400714 (CN)
(74) Representative: Rigamonti, Dorotea
(86) International application number: PCT/CN2024/074211
(87) International publication number: WO 2024/169567

(57) **Abstract**

Provided are an arginine deiminase mutant, a covalent dimer and a conjugate and a use thereof. The provided arginine deiminase mutant forms a covalent disulfide bond between arginine deiminase subunits by means of mutation of an amino acid site on arginine deiminase. Also provided are a covalent conjugate and a use in the field of cancer treatment. Site-directed mutagenesis of an amino acid site promotes formation of a covalent disulfide bond between non-covalent homodimeric arginine deiminase subunits, and a formed dimer has a more stable structure; in addition, the exposure level of an epitope on an interaction interface can be reduced, the in vivo immunogenicity is reduced, and a more excellent anti-tumor effect is exhibited.

## Description

### PRIORITY INFORMATION

The present disclosure claims priority to and benefits of patent application No. 202310115527.8, filed with China National Intellectual Property Administration on February 15, 2023, the entire contents of which are incorporated herein by reference.

### FIELD

The present disclosure relates to the field of biomedicine, and specifically to an arginine deiminase mutant, a covalent dimer, a conjugate and a use thereof, in particular to use of a conjugate, especially a PEGylated covalent dimeric arginine deiminase mutant, in the manufacture of a medicament for arginine depletion, which reduces in vivo arginine levels, prolongs the in vivo half-life and reduces the immunogenicity.

### BACKGROUND

Amino acid deprivation therapy can serve as an effective treatment for some metabolic diseases or specific types of cancer. So far, based on this therapy, some well-known cases involving marketed drugs include: the use of an L-covalent dimeric arginine deiminase mutant to reduce blood asparagine levels in the treatment of acute lymphoblastic leukemia, and the use of phenylalanine ammonia lyase (PAL) to reduce blood phenylalanine (Phe) levels in adult patients with phenylketonuria (PKU). Arginine deprivation therapy represents another promising approach for controlling certain cancers such as hepatocellular carcinoma and melanoma. Certain tumor cells exhibit metabolic pathways distinct from those of normal cells. Studies have shown that arginine is a non-essential amino acid for humans (Rogers, 1994), and normal cells are capable of synthesizing arginine endogenously by converting citrulline through argininosuccinate synthetase (ASS) and argininosuccinate lyase (ASL) (Haines, 2011). However, in tumors such as hepatocellular carcinoma, melanoma, and certain sarcomas, arginine becomes an essential amino acid due to deficiencies in one or both of these enzymes. These tumors rely on extracellular arginine for survival and are thus considered auxotrophic for arginine. Two main enzymes have been extensively studied for use in arginine deprivation therapy against cancers such as liver cancer, melanoma, and renal cell carcinoma: arginase and arginine deiminase (ADI).

For arginase, human Type 1 arginase is currently applied in clinical research. However, its clinical use is limited by low catalytic activity in serum and high immunogenicity. To address these issues, Aeglea Biotherapeutics substituted cobalt ions for manganese ions to enhance arginase's enzymatic activity and stability in serum, and employed PEGylation on arginase to reduce immunogenicity. This modified arginase is currently under clinical investigation.

Arginine deiminase (ADI, EC 3.5.3.6) is another microbial enzyme capable of degrading arginine into citrulline and ammonia. Arginine deiminases from different sources vary in structure and physiological activity. For example, arginine deiminase extracted from *Pseudomonas putida* has shown effective in vitro cytotoxicity against cancer cells (Jones JB, in 1981), but lacks activity under physiological pH conditions, limiting its in vivo application. Currently, structural modifications have been reported that are active under physiological pH conditions. In contrast, arginine deiminase derived from *Mycoplasma* exhibits high activity under physiological conditions (Takaku, 1992).

As a therapeutic drug, arginine deiminase shares common limitations with other protein drugs, such as poor stability, susceptibility to degradation, and short in vivo half-life, which is only about 4 h in mice. In addition, arginine deiminase is also an exogenous protein with high immunogenicity, inducing the organism to produce antibodies that cause enzymes to become inactive or immune-related adverse effects. Chemical modifications (such as using PEG, fatty acids, and polysaccharides) on proteins can effectively shield immunogenic epitopes, reduce immunogenicity, and increase the molecular weight of the protein, thereby improving in vivo half-life. Therefore, chemical modifications on protein drugs such as using PEG, dextran, and peptide repeat sequence (PAS) are currently effective methods for reducing the immunogenicity. Polyethylene glycol (PEG) is a linear or branched polyether polymer formed by the polymerization of ethylene oxide. Due to its excellent hydrophilicity, biocompatibility, and biological inertness, PEG has been widely used to modify protein drugs and can significantly reduce immunogenicity and prolong in vivo half-life. PEGylation of drugs is considered one of the most effective techniques in protein drug development, and has been very mature in clinical practice. Covalent conjugation of PEG and arginine deiminase facilitates shielding epitopes on the surface, reducing the immunogenicity, and prolonging the in vivo half-life. At present, the most extensively studied research is the arginine deiminase derived from *Mycoplasma hominis* and modified with SS-PEG-20K, which has entered clinical research (ADI-PEG-20). However, due to the presence of unstable ester bonds in its structure, polyethylene glycol can easily detach in blood, leading to re-exposure of antigens on ADI's surface and incomplete resolution of immunogenicity. Moreover, the native ADI from *Mycoplasma hominis* is in the form of a homodimer stabilized by non-covalent interactions such as hydrogen bonds and hydrophobic forces, which makes it structurally unstable and poor in vivo stability. When PEGylated, it has an unstable modified structure and may dissociate in vivo, further exposing epitope at the interfaces, which leads to high immunogenicity and low enzyme activity retention of the drug.

Therefore, how to improve the stability of arginine deiminase and reduce the immunogenicity of arginine deiminase in vivo remains major technical challenges in this field.

### SUMMARY

The present disclosure aims to solve one of the technical problems in the related art at least to a certain extent. To this end, an object of the present disclosure is to provide a covalent dimeric arginine deiminase mutant, and a conjugate and a use thereof. By performing site-directed mutagenesis on an amino acid site, an inter-subunit covalent disulfide bond is formed between subunits of the non-covalent homodimeric arginine deiminase, enabling an enhanced structural stability of the formed dimer and reduced exposure of epitopes at the interface, thereby reducing immunogenicity in vivo.

Specifically, the present disclosure provides the following technical solutions.

In a first aspect of the present disclosure, an arginine deiminase mutant is provided. The mutant comprises an inter-subunit covalent disulfide bond formed between arginine deiminase subunits via mutation at an amino acid site on arginine deiminase.

The arginine deiminase mutant may further include the following technical features.

According to the arginine deiminase mutant, the mutant exists in the form of a covalent dimer, the covalent dimer formed between non-covalent homodimeric arginine deiminase subunits by site-directed mutagenesis at an amino acid site on arginine deiminase.

According to the arginine deiminase mutant, based on a sequence derived from *Mycoplasma hominis* or a homologous sequence thereof, at least one amino acid at positions 46, 145 and 337 of a sequence as set forth in SEQ ID NO: 1 is mutated to cysteine in the mutant.

According to the arginine deiminase mutant, the sequence derived from *Mycoplasma hominis* or the homologous sequence thereof is the sequence as set forth in SEQ ID NO: 1, or a sequence having at least 80%, 81%, 82%, 83%, 84%, 85%, 86%, 87%, 88%, 89%, 90%, 91%, 92%, 93%, 94%, 95%, 96%, 97%, 98%, or 99% homology to the sequence as set forth in SEQ ID NO: 1.

According to the arginine deiminase mutant, the mutant is derived from *Mycoplasma hominis,* an amino acid at position 46 of a sequence as set forth in SEQ ID NO: 1 is mutated to cysteine, and an amino acid sequence of the arginine deiminase subunit of the mutant is as set forth in SEQ ID NO: 2.

According to the arginine deiminase mutant, the mutant is derived from *Mycoplasma hominis,* an amino acid at position 145 of a sequence as set forth in SEQ ID NO: 1 is mutated to cysteine, and an amino acid sequence of the arginine deiminase subunit of the mutant is as set forth in SEQ ID NO: 3.

According to the arginine deiminase mutant, the mutant is derived from *Mycoplasma hominis,* an amino acid at position 337 of a sequence as set forth in SEQ ID NO: 1 is mutated to cysteine, and an amino acid sequence of the arginine deiminase subunit of the mutant is as set forth in SEQ ID NO: 4.

In a second aspect of the present disclosure, a covalent dimer is provided. The covalent dimer includes the above arginine deiminase mutant.

In a third aspect of the present disclosure, a covalent conjugate is provided. The covalent conjugate includes the above arginine deiminase mutant or covalent dimer and a modifying group coupled thereto. The modifying group forms an amide bond with ε-amino of a lysine residue or α-amino of an N-terminal amino acid residue of the arginine deiminase mutant.

According to an embodiment of the present disclosure, the covalent conjugate may further include the following technical features.

According to an embodiment of the present disclosure, the modifying group is selected from at least one of polyethylene glycol, dextran, and a peptide repeat sequence, having an active group.

According to an embodiment of the present disclosure, the modifying group has a molecular weight of 2 KDa to 20 KDa, preferably 5 KDa.

According to an embodiment of the present disclosure, the number of the modifying group is from 9 to 20.

According to an embodiment of the present disclosure, the active group is selected from at least one of succinimidyl carbonate, succinimidyl propionate, succinimidyl acetate, and succinimidyl succinate, preferably succinimidyl propionate.

According to a preferred embodiment of the present disclosure, the modifying group is polyethylene glycol having an active group.

According to a preferred embodiment of the present disclosure, the polyethylene glycol is linear or branched polyethylene glycol, preferably linear.

According to an embodiment of the present disclosure, the polyethylene glycol has a molecular weight of 2 KDa to 20 KDa, preferably 5 KDa.

According to an embodiment of the present disclosure, the active group is selected from at least one of succinimidyl carbonate, succinimidyl propionate, succinimidyl acetate, and succinimidyl succinate, preferably succinimidyl propionate.

According to an embodiment of the present disclosure, the covalent conjugate exhibits at least one of the following properties:
(a) the conjugate has an enzyme activity retention rate of not less than 50% that of the arginine deiminase mutant in the form of a covalent dimer;
(b) the conjugate has low immunogenicity and prolonged in vivo half-life; and
(c) the conjugate is capable of reducing an in vivo arginine level.

In a fourth aspect of the present disclosure, a pharmaceutical composition is provided. The pharmaceutical composition includes the above arginine deiminase mutant, covalent dimer, or covalent conjugate, and a pharmaceutically acceptable carrier.

In a fifth aspect of the present disclosure, use of the arginine deiminase mutant in the first aspect, the covalent dimer in the second aspect, the covalent conjugate in the third aspect, or the pharmaceutical composition in the fourth aspect in the manufacture of a medicament for the treatment of a tumor is provided.

According to an embodiment of the present disclosure, the use may further include the following technical features.

According to an embodiment of the present disclosure, the tumor is an arginine-dependent tumor.

According to an embodiment of the present disclosure, the tumor is ASS-deficient, P53 wild-type, or a combination thereof.

According an embodiment of the present disclosure, the tumor is selected from at least one of hepatocellular carcinoma, melanoma, pancreatic cancer, colorectal cancer, acute myeloid leukemia, and small cell lung cancer.

In a sixth aspect of the present disclosure, use of the arginine deiminase mutant in the first aspect, the covalent dimer in the second aspect, the covalent conjugate in the third aspect, or the pharmaceutical composition in the fourth aspect in the prevention or treatment of a tumor is provided.

According to an embodiment of the present disclosure, the tumor is an arginine-dependent tumor.

According to an embodiment of the present disclosure, the tumor is ASS-deficient, P53 wild-type, or a combination thereof.

According an embodiment of the present disclosure, the tumor is selected from at least one of hepatocellular carcinoma, melanoma, pancreatic cancer, colorectal cancer, acute myeloid leukemia, and small cell lung cancer.

In a seventh aspect of the present disclosure, a method for preventing or treating a tumor is provided. The method includes: administering to a subject at least one of the followings:
the arginine deiminase mutant in the first aspect;
the covalent dimer in the second aspect;
the covalent conjugate in the third aspect; or
the pharmaceutical composition in the fourth aspect.

According to an embodiment of the present disclosure, the tumor is an arginine-dependent tumor.

According to an embodiment of the present disclosure, the tumor is ASS-deficient, P53 wild-type, or a combination thereof.

According an embodiment of the present disclosure, the tumor is selected from at least one of hepatocellular carcinoma, melanoma, pancreatic cancer, colorectal cancer, acute myeloid leukemia, and small cell lung cancer.

The additional aspects and advantages of the present disclosure will be partially given in the following description, partially apparent from the following description, or be learned through the practice of the present disclosure.

### BRIEF DESCRIPTION OF DRAWINGS

The above and/or additional aspects and advantages of the present disclosure will become obvious and easy to understand from the description of the embodiments in conjunction with the following accompanying drawings.
FIG. 1 shows the SDS-PAGE analysis of ADI or mADI expression induced by IPTG according to an embodiment of the present disclosure. From left to right, Lane 1: Marker; Lane 2: AWT; Lane 3: A1C; Lane 4: A2C; Lane 5: A3C. The molecular weights (kDa) of the marker from top to bottom are 94.0, 66.2, 45.0, 33.0, 26.0, 20.0, and 14.4.
FIG. 2 shows the SDS-PAGE analysis after purification of ADI or mADI according to an embodiment of the present disclosure. From left to right, Lane 1: A1C; Lane 2: Marker; Lane 3: AWT. The molecular weights (kDa) of the marker from top to bottom are 97.4, 66.2, 43.0, 31.0, 22.0, and 14.4.
FIG. 3 shows a comparison of SEC-HPLC chromatograms after purification of ADI or mADI according to an embodiment of the present disclosure. The retention time of the main peak at 26.577 min corresponds to A1C, and at 29.693 min corresponds to AWT.
FIG. 4 shows the SEC-HPLC chromatogram of PEG-AlC-5K after purification according to an embodiment of the present disclosure.
FIG. 5 shows the plasma concentration-time curve in rats after a single dose administration of PEG-A1C-5K or PEG-AWT-5K according to an embodiment of the present disclosure.
FIG. 6 shows the plasma concentration-time curve in rats after multiple dose administrations of PEG-A1C-5K or PEG-AWT-5K according to an embodiment of the present disclosure.
FIG. 7 shows the plasma concentration-time curve in rhesus monkeys after a single dose administration of PEG-A1C-5K according to an embodiment of the present disclosure.
FIG. 8 shows the plasma concentration-time curve in rhesus monkeys after multiple dose administrations of PEG-A1C-5K according to an embodiment of the present disclosure.
FIG. 9 shows the tumor volume-time curve in melanoma-bearing mice with different treatment groups according to an embodiment of the present disclosure.
FIG. 10 shows the tumor weight-time curve in melanoma-bearing mice with different treatment groups.
FIG. 11 shows a comparison of serum arginine concentrations in vivo among different treatment groups after the final dose in melanoma-bearing mice according to an embodiment of the present disclosure.
FIG. 12 shows a comparison of serum citrulline concentrations in vivo among different treatment groups after the final dose in melanoma-bearing mice according to an embodiment of the present disclosure.

### DETAILED DESCRIPTION

The embodiments of the present disclosure are described in detail below. The embodiments described below are exemplary, and are only intended to explain the present disclosure, rather than being construed as limitations to the present disclosure.

Moreover, the terms "first" and "second" are only for the purpose of description and should not be construed as indicating or implying relative importance or implicitly indicating the number of technical features indicated. Thus, the features defined by the terms "first" and "second" may include one or more of the features either explicitly or implicitly. In the descriptions of the present disclosure, unless otherwise specifically limited, the term "plurality" means at least two, such as two, three, etc.

As used herein, the terms "arginine deiminase mutant", "arginine deiminase analogue", "arginine deiminase derivative", and "covalent dimeric arginine deiminase mutant" may be used interchangeably in certain contexts, and refer to proteins that retain the specific catalytic activity of arginine deiminase (i.e., converting arginine into citrulline and ammonia), and are structurally modified on the basis of the native arginine deiminase protein sequence by substitution, deletion, or addition of one or more amino acid residues. Such modifications achieve, for example, stabilization of the protein structure, maintenance of enzymatic activity, and reduction of immunogenicity, as exemplified in the present disclosure.

The terms "modifier", "modifying group", and "polymer" used in the present disclosure may be used interchangeably, and refer to polymers used for the modifications of proteins and polypeptide drugs, such as polyethylene glycol.

The terms "conjugate", "coupled product", and "modified product" used in the present disclosure may be used interchangeably, and refer to a modified product that is obtained by modifying arginine deiminase or its mutant with a modifying group.

The terms "PEG" and "polyethylene glycol" used in the present disclosure refers to a linear polymer with hydroxyl groups at both ends (HO-(CH₂CH₂O)n-CH₂CH₂OH), which may be branched, linear, or multi-arm. Conventional polyethylene glycol has hydroxyl groups at both ends, and when one end is capped with a methyl group, the resultant is methoxy polyethylene glycol (mPEG), which is most commonly used in protein PEGylation technologies. PEG may be further functionalized to introduce active groups for conjugation.

The terms "covalent dimeric arginine deiminase mutant conjugate", " PEGylated arginine deiminase mutant", and " PEGylated arginine deiminase" may be used interchangeably in certain contexts, and refer to a conjugate which is formed by modifying arginine deaminase or its mutant with a modifying group such as active polyethylene glycol.

The terms "average degree of modification ", "average number of modifications", "average number of conjugations", and "average number of modifying group" may be used interchangeably, and refer to the number of PEGs conjugated per arginine deiminase subunit.

It should be noted that the molecular weight expressed by "PEG molecular weight" or "PEG relative molecular weight" in the present application has general sense in this field. For example, the total relative molecular weight of PEGs which are activated by an active group is expressed as 5 KDa within the range of 5 KDa + 10%.

As described herein, for convenience, the wild-type non-covalent dimeric arginine deiminase is represented by ADI or AWT, while the covalent dimeric arginine deiminase mutant is collectively represented by mADI, and A1C, A2C, A3C and the like represent specific mutation types of mADI.

In an aspect of the present disclosure, an arginine deiminase mutant is provided. The mutant forms an inter-subunit covalent disulfide bond between arginine deiminase subunits via mutation at an amino acid site on arginine deiminase. Through mutation at an amino acid site on arginine deiminase, an inter-subunit covalent disulfide bond is formed, either between a mutation site and another mutation site, or between a mutation site and a cysteine residue on another arginine deiminase subunit. As a result, a covalent dimer is formed.

According to a specific embodiment of the present disclosure, the mutant promotes the formation of a covalent dimer between non-covalent homodimeric arginine deiminase subunits by site-directed mutagenesis at an amino acid site on arginine deiminase.

According to a specific embodiment of the present disclosure, the mutant is derived from *Mycoplasma hominis* or a homologous sequence thereof. The mutation site is selected from at least one of position 46 (wild-type amino acid A), position 145 (wild-type amino acid E), and position 337 (wild-type amino acid E) of the sequence as set forth in SEQ ID NO: 1, and the corresponding amino acid(s) are mutated to cysteine. A stable inter-subunit covalent disulfide bond is formed, either between identical mutation sites across subunits (e.g., C46-C46, C145-C145, or C337-C337), or between a mutation site on one subunit and a different cysteine residue on another subunit (e.g., C46-C396, C145-C396, or C337-C396). As a result, a stable covalent dimer is formed. The sequence as set forth in SEQ ID NO: 1 corresponds to an arginine deiminase derived from *Mycoplasma hominis.* Those skilled in the art understand that homologous sequences may differ from SEQ ID NO: 1. According to a specific embodiment of the present disclosure, the sequence derived from *Mycoplasma hominis* or the homologous sequence thereof is the sequence as set forth in SEQ ID NO: 1, or a sequence having at least 80%, 81%, 82%, 83%, 84%, 85%, 86%, 87%, 88%, 89%, 90%, 91%, 92%, 93%, 94%, 95%, 96%, 97%, 98%, or 99% homology to the sequence as set forth in SEQ ID NO: 1. According to a specific embodiment, the mutation site(s) of the provided mutant are selected from at least one of position 46, 145, or 337 of a sequence as set forth in SEQ ID NO: 1, with the respective amino acid(s) mutated to cysteine. While SEQ ID NO: 1 shows the sequence derived from *Mycoplasma hominis,* those skilled in the art understand that homologous sequences may differ from SEQ ID NO: 1, and it is impractical to enumerate all such sequences. However, any mutant in which the corresponding positions 46, 145, and 337 or equivalent positions in SEQ ID NO:1 or homologous sequences are mutated as described above shall be contemplated. The "equivalent positions" mentioned refer to amino acids in the homologous sequence that perform the same or similar function as the position 46, 145, or 337, which may be at positions 37, 146, 338 or others. Details may refer to the contents listed in Tables 1 and 2 below.

According to a specific embodiment of the present disclosure, the mutant is derived from other *Mycoplasma* species, and the mutation site correspond to at least one of positions 46, 145, and 337 in the sequence as set forth in SEQ ID NO: 1, with the respective amino acid(s) mutated to cysteine.

It should be understood that arginine deiminases derived from other organisms or pathways such as structural modifications may also have a mutation at a position corresponding to that of arginine deiminase derived from *Mycoplasma hominis.* For example, Table 1 below shows the comparison of sequence homology across arginine deiminases derived from different organisms, including *Mycoplasma phocicerebrale, Mycoplasma arginini, Mycoplasma arthritidis,* etc. Their amino acid sequences have at least 80% homology to that of *Mycoplasma hominis,* and have similarity in a spatial structure. The amino acid mutation or difference on the surface does not affect the reduction of the enzyme activity of arginine. A person skilled in the art may obtain the covalent dimer by implementing well-known techniques in the art. For example, it can be achieved by mutating at least one of amino acids at corresponding positions 46, 145, and 337 in the sequence as set forth in SEQ ID NO: 1 into cysteine at the mutation site. When arginine deiminase is derived from different sources, the mutation site may differ accordingly, but all these changes are within the protection scope of the present disclosure. According to a specific embodiment of the present disclosure, the specific mutation sites are shown in Table 2, which shows the comparison of mutation sites of arginine deiminase from different sources.

**Table 1: Homology Comparison Across Arginine Deiminase from Various Organisms**

| Source | Amino Acid Sequence | Homology |
|---|---|---|
| *Mycoplasma phocicerebrale* | IHVYSEIGELETVLVHEPGREIDYITPARLDELLFSAILESHDARKEH QSFVKQLKDNGINVVELTDLVAETFDLASKEEQEKLIEEFLEDSEPV LSEAHKTAVRKFLTSRKSTREMVEFMMAGITKYDLGIEADHELIVD PMPNLYFTRDPFASVGNGVTIHYMRYKVRQRETLFSRFVFSNHPKL VKTPWYYDPAMKMSIEGGDVFIYNNDTLVVGVSERTDLETITLLAK NIKANKEVEFKRIVAINVPKWTNLMHLDTWLTMLDKDKFLYSPIAN DVFKFWDYDLVNGGAEPQPKENGLPLEGLLQSIINKKPVLIPIAGNN ASHIDIERETHFDGTNYLAIKPGVVIGYARNEKTNAALAAAGIKVLP FHGNQLSLGMGNARCMSMP (SEQ ID NO: 9) | 82.1% |
| *Mycoplasma arginini* | SVFDSKFKGIHVYSEIGELESVLVHEPGREIDYITPARLDELLFSAILE SHDARKEHKQFVAELKANDINVVELIDLVAETYDLASQEAKDKLIE EFLEDSEPVLSEEHKVVVRNFLKAKKTSRELVEIMMAGITKYDLGI EADHELIVDPMPNLYFTRDPFASVGNGVTIHYMRYKVRQRETLFSR FVFSNHPKLINTPWYYDPSLKLSIEGGDVFIYNNDTLVVGVSERTDL QTVTLLAKNIVANKECEFKRIVAINVPKWTNLMHLDTWLTMLDKD KFLYSPIANDVFKFWDYDLVNGGAEPQPVENGLPLEGLLQSIINKKP VLIPIAGEGASQMEIERETHFDGTNYLAIRPGVVIGYSRNEKTNAAL EAAGIKVLPFHGNQLSLGMGNARCMSMPLSRKDVKW (SEQ ID NO: 10) | 82.1% |
| *Mycoplasma arthritidis* | SVFDSKFKGIHVYSEIGELETVLVHEPGKEIDYITPARLDELLFSAILE SHDARKEHKEFVAELKKRGINVVELVDLIVETYDLASKEAKEKLLE EFLDDSAPVLSDEHRAAVKKFLQSQKSTRSLVEYMIAGITKHDLKIE SDLELIVDPMPNLYFTRDPFASVGNGVTIHYMRYKVRQRETLFSRF VFSNHPKLVNTPWYYDPAEGLSIEGGDVFIYNNDTLVVGVSERTDL QTITLLAKNIKANKECEFKRIVAINVPKWTNLMHLDTWLTMLDKD KFLYSPIANDVFKFWDYDLVNGGDAPQPVDNGLPLEDLLKSIIGKK PTLIPIAGAGASQIDIERETHFDGTNYLAVAPGIVIGYARNEKTNAAL EAAGITVLPFRGNQLSLGMGNARCMSMPLSRKDVK (SEQ ID NO: 11) | 80.4% |

**Table 2: Comparison of Mutation Sites in Arginine Deiminase from Different Sources**

| Source | Mutation Site 1 | Mutation Site 2 | Mutation Site 3 |
|---|---|---|---|
| *Mycoplasma hominis* | A46 | E145 | E337 |
| *Mycoplasma phocicerebrale* | A37 | E137 | H329 |
| *Mycoplasma arginini* | A46 | E146 | Q338 |
| *Mycoplasma arthritidis* | A46 | E146 | Q338 |

According to a specific embodiment of the present disclosure, with *Mycoplasma hominis* as the source, an amino acid mutation site of the provided mutant is mutated into cysteine from the amino acid at position 46 in the sequence as set forth in SEQ ID NO: 1, and the amino acid sequence of the formed arginine deiminase subunit is as set forth in SEQ ID NO: 2. According to a specific embodiment of the present disclosure, with *Mycoplasma hominis* as the source, an amino acid mutation site of the provided mutant is mutated into cysteine from the amino acid at position 145 in the sequence as set forth in SEQ ID NO: 1, and the amino acid sequence of the formed arginine deiminase subunit is as set forth in SEQ ID NO: 3. According to a specific embodiment of the present disclosure, with *Mycoplasma hominis* as the source, an amino acid mutation site of the provided mutant is mutated into cysteine from the amino acid at position 337 in the sequence as set forth in SEQ ID NO: 1, and the amino acid sequence of the formed arginine deiminase subunit is as set forth in SEQ ID NO: 4.

According to a specific embodiment of the present disclosure, with *Mycoplasma hominis* as the source, an amino acid mutation site of the mutant is mutated into cysteine from the amino acid at position 46 in the sequence as set forth in SEQ ID NO: 1. According to a specific embodiment of the present disclosure, with *Mycoplasma hominis* as the source, an amino acid mutation site of the mutant is mutated into cysteine from the amino acid at position 145 in the sequence as set forth in SEQ ID NO: 1. According to a specific embodiment of the present disclosure, with *Mycoplasma hominis* as the source, an amino acid mutation site of the mutant is mutated into cysteine from the amino acid at position 337 in the sequence as set forth in SEQ ID NO: 1. The provided mutant includes any combination of two or all three of the above mutation sites. The present disclosure further provides a method for producing a covalent dimeric arginine deiminase mutant, which enhances the structural stability of the arginine deiminase dimeric mutant and reduces the exposure of epitopes at the interface.

**Table 3: Sequence Information**

| SEQ ID NO: | Sequence Information |
|---|---|
| 1 | SVFDSKFNGIHVYSEIGELETVLVHEPGREIDYITPARLDELLFSAILESHDARKEHQSFV KIMKDRGINVVELTDLVAETYDLASKAAKEEFIETFLEETVPVLTEANKKAVRAFLLSK PTHEMVEFMMSGITKYELGVESENELIVDPMPNLYFTRDPFASVGNGVTIHFMRYIVR RRETLFARFVFRNIIPKLVKTPWYYDPAMKMPIEGGDVFIYNNETLVVGVSERTDLDTI TLLAKNIKANKEVEFKRIVAINVPKWTNLMHLDTWLTMLDKNKFLYSPIANDVFKFW DYDLVNGGAEPQPQLNGLPLDKLLASIINKEPVLIPIGGAGATEMEIARETNFDGTNYL AIKPGLVIGYDRNEKTNAALKAAGITVLPFHGNQLSLGMGNARCMSMPLSRKDVKW |
| 2 | SVFDSKFNGIHVYSEIGELETVLVHEPGREIDYITPARLDELLFSCILESHDARKEHQSFV KIMKDRGINVVELTDLVAETYDLASKAAKEEFIETFLEETVPVLTEANKKAVRAFLLSK PTHEMVEFMMSGITKYELGVESENELIVDPMPNLYFTRDPFASVGNGVTIHFMRYIVR RRETLFARFVFRNHPKLVKTPWYYDPAMKMPIEGGDVFIYNNETLVVGVSERTDLDTI TLLAKNIKANKEVEFKRIVAINVPKWTNLMHLDTWLTMLDKNKFLYSPIANDVFKFW DYDLVNGGAEPQPQLNGLPLDKLLASIINKEPVLIPIGGAGATEMEIARETNFDGTNYL AIKPGLVIGYDRNEKTNAALKAAGITVLPFHGNQLSLGMGNARCMSMPLSRKDVKW |
| 3 | SVFDSKFNGIHVYSEIGELETVLVHEPGREIDYITPARLDELLFSAILESHDARKEHQSFV KIMKDRGINVVELTDLVAETYDLASKAAKEEFIETFLEETVPVLTEANKKAVRAFLLSK PTHEMVEFMMSGITKYELGVESENCLIVDPMPNLYFTRDPFASVGNGVTIHFMRYIVR RRETLFARFVFRNHPKLVKTPWYYDPAMKMPIEGGDVFIYNNETLVVGVSERTDLDTI TLLAKNIKANKEVEFKRIVAINVPKWTNLMHLDTWLTMLDKNKFLYSPIANDVFKFW DYDLVNGGAEPQPQLNGLPLDKLLASIINKEPVLIPIGGAGATEMEIARETNFDGTNYL AIKPGLVIGYDRNEKTNAALKAAGITVLPFHGNQLSLGMGNARCMSMPLSRKDVKW |
| 4 | SVFDSKFNGIHVYSEIGELETVLVHEPGREIDYITPARLDELLFSAILESHDARKEHQSFV KIMKDRGINVVELTDLVAETYDLASKAAKEEFIETFLEETVPVLTEANKKAVRAFLLSK PTHEMVEFMMSGITKYELGVESENELIVDPMPNLYFTRDPFASVGNGVTIHFMRYIVR RRETLFARFVFRNHPKLVKTPWYYDPAMKMPIEGGDVFIYNNETLVVGVSERTDLDTI TLLAKNIKANKEVEFKRIVAINVPKWTNLMHLDTWLTMLDKNKFLYSPIANDVFKFW DYDLVNGGAEPQPQLNGLPLDKLLASIINKEPVLIPIGGAGATCMEIARETNFDGTNYL AIKPGLVIGYDRNEKTNAALKAAGITVLPFHGNQLSLGMGNARCMSMPLSRKDVKW |

The present disclosure further provides a covalent dimer. The covalent dimer includes the above-mentioned arginine deiminase mutant.

In another aspect of the present disclosure, a method for preparing a covalent dimeric arginine deiminase mutant is provided.

In another preferred embodiment, the covalent dimeric arginine deiminase mutant of the present disclosure, derived from different species or from sources having at least 80% homology with the same species, may be obtained through a variety of pathways, including but not limited to chemical synthesis, genetically engineered recombinant expression, etc.

In another preferred example, the mutant is prepared by constructing a recombinant expression strain with *Escherichia coli* or yeast as a host, preferably *Escherichia coli.*

The covalent dimeric arginine deiminase mutant of the present disclosure may be recombinantly expressed in *Escherichia coli* to obtain a large number of the arginine deiminase mutant. The expressed arginine deiminase mutant may be expressed in cells, on the cell membrane, or secreted to the outside of cells. If necessary, high-purity covalent dimeric arginine deiminase mutant may be obtained using methods known to a person skilled in the art. Examples of these methods include, but are not limited to: centrifugation, bacterial disruption, salt precipitation, ultrafiltration, ion exchange chromatography, hydrophobic chromatography, molecular sieve chromatography, or a combination of these techniques.

The present disclosure further provides a covalent conjugate. The covalent conjugate includes the arginine deiminase mutant or covalent dimer, and a modifying group chemically coupled thereto. Chemical coupling can reduce the in vivo immunogenicity and extend the in vivo half-life.

According to a specific embodiment of the present disclosure, the modifying group forms an amide bond with ε-amino of a lysine residue or α-amino of an N-terminal amino acid residue of the arginine deiminase mutant.

According to a specific embodiment of the present disclosure, the number of the modifying group is from 9 to 20. The modifying group is at least one of polyethylene glycol, dextran, and a peptide repeat sequence, having an active group. According to a preferred embodiment of the present disclosure, the modifying group is polyethylene glycol.

According to a specific embodiment of the present disclosure, the modifying group has a molecular weight of 2 KDa to 20 KDa. According to a preferred embodiment of the present disclosure, the modifying group has a molecular weight of 5 KDa.

According to a specific embodiment of the present disclosure, the active group is selected from at least one of succinimidyl carbonate, succinimidyl propionate, succinimidyl acetate, and succinimidyl succinate.

According to a specific embodiment of the present disclosure, the modifying group forms an amide bond with ε-amino of a lysine residue or α-amino of an N-terminal amino acid residue of the arginine deiminase mutant. The number of the modifying group is from 9 to 20. The modifying group is polyethylene glycol having an active group. According to a specific embodiment of the present disclosure, the polyethylene glycol (PEG) is linear or branched polyethylene glycol, preferably linear polyethylene glycol. For example, the polyethylene glycol is capped with a blocking group at one end, and the blocking group includes, but is not limited to, at least one of monomethoxyl, ethoxyl, glucose, or galactose, for example preferably, monomethoxyl.

According to a specific embodiment of the present disclosure, the polyethylene glycol has a molecular weight of 2 KDa to 20 KDa, preferably 5 KDa.

According to a specific embodiment of the present disclosure, an active group of polyethylene glycol is selected from at least one of succinimidyl carbonate, succinimidyl propionate, succinimidyl acetate, and succinimidyl succinate. For example, the active group may be succinimidyl propionate.

According to a specific embodiment of the present disclosure, the covalent conjugate has at least one of the following features:
(a) an amino acid sequence of the covalent dimeric arginine deiminase mutant subunit is as set forth in SEQ ID NO: 2;
(B) a modifying group of the conjugate is randomly coupled to at least one, preferably 9-20 modifying groups by forming an amide bond with ε-amino of a lysine residue or α-amino of an N-terminal amino acid residue in the covalent dimeric arginine deiminase mutant;
(c) the modifying group is polyethylene glycol having an active group;
(d) the polyethylene glycol is linear polyethylene glycol;
(e) the polyethylene glycol is capped with monomethoxyl at one end;
(f) the polyethylene glycol has a molecular weight of 5 KDa;
(g) the active group is succinimidyl propionate; and
(h) the conjugate is a PEGylated arginine deiminase mutant, and the obtained pegylated arginine deiminase mutant has the characteristics of enzyme activity retention rate of not less than 50% of that of the covalent dimeric arginine deiminase mutant, stable structure, low immunogenicity, and prolonged in vivo half-life.

The arginine deiminase mutant modified with polyethylene glycol may be prepared by methods commonly used in the art. The samples may be purified by methods known in the art. These methods include, but are not limited to, molecular sieve chromatography, ion exchange chromatography, hydrophobic chromatography, ultrafiltration, or combinations thereof. Molecular sieve chromatography and ultrafiltration are preferred.

Another object of the present disclosure is to provide use of an arginine deiminase mutant or a conjugate thereof. The conjugate has the characteristics of enzyme activity retention rate of not less than 50% of that of the covalent dimeric arginine deiminase mutant, stable structure, low immunogenicity, and prolonged in vivo half-life, and can be used for arginine depletion alone or in combination, for the treatment of arginine-dependent tumors.

The present disclosure further provides a pharmaceutical composition. The pharmaceutical composition includes the above-mentioned arginine deiminase mutant, covalent dimer, or covalent conjugate, and a pharmaceutically acceptable carrier. The pharmaceutically acceptable carrier includes any and all physiologically compatible solvents, dispersion media, coatings, antibacterial and antifungal agents, isotonic and absorption retardants, etc. Preferably, the carrier is suitable for intravenous, intramuscular, subcutaneous, parenteral, spinal or epidermal administration (e.g., by injection or infusion). Depending on different dosing routes, an active compound, i.e., an antibody, may be encapsulated in a material to protect this compound from acidic effects and other natural conditions that may inactivate this compound. The provided pharmaceutical composition may be administrated by many methods known in the art. The antibody may be prepared together with suitable carriers, such as controlled-release preparations, including implants, transdermal patches, and microencapsulated delivery systems. Biodegradable, biocompatible polymers such as ethylene vinyl acetate, polyanhydride, polyglycolic acid, collagen, polyorthoesters and polylactic acid may be used. Many methods used to prepare such preparations have been patented or are generally known to a person skilled in the art.

The present disclosure further provides a method for treating a disease. The method includes: administering to a subject in need a therapeutically effective amount of the above-mentioned arginine deiminase mutant, covalent dimer, covalent conjugate or pharmaceutical composition. The mentioned mutant, covalent dimer or covalent conjugate may be used for arginine depletion alone in the form of salts or other pharmaceutical compositions, for the treatment of arginine-dependent tumors.

According to a specific embodiment of the present disclosure, the conjugate is used for the treatment of arginine-dependent tumors, and the tumor is ASS-deficient, P53 wild-type, or a combination thereof. The mentioned tumors include, but are not limited to, hepatocellular carcinoma, melanoma, pancreatic cancer, colorectal cancer, acute myeloid leukemia, small cell lung cancer, etc.

The dosing frequency and dosage of the conjugate or other pharmaceutical compositions of the present disclosure may be determined by a plurality of relevant factors, including a type of a disease to be treated, dosing route, patient's age, gender and weight and severity of the disease, as well as a type of a drug used as an active ingredient.

The term "treatment" is used for obtaining a desired pharmacological and/or physiological effect. Said effect may be prophylactic in terms of complete or partial prevention of diseases or their symptoms, and/or may be therapeutic in terms of partial or complete cure of a disease and/or adverse effects caused by the disease. The term "treatment" as used herein encompasses diseases of mammals, particularly humans, including: (a) prevention of a disease in individuals who are susceptible to disease but have not yet been diagnosed definitely; (b) inhibition of a disease, e.g., arresting the development of the disease; or (c) alleviation of a disease, e.g., mitigating symptoms associated with the disease. The "treatment" as used herein encompasses any medication in which a drug or compound is administered to an individual to treat, cure, alleviate, improve, mitigate, or suppress an individual's disease, including, but not limited to, administrating to an individual in need the covalent dimeric arginine deiminase mutant conjugate described herein.

"Therapeutically effective amount" or "effective amount" means the amount of the mutant, covalent dimer or pharmaceutical composition required to elicit the desired biological response. According to the present disclosure, "therapeutically effective amount" means the amount of the mutant, covalent dimer or pharmaceutical composition required to treat and/or prevent a disease.

"Therapeutically effective amount" as used herein may be easily determined by a person of ordinary skill in the art depending on the relative activity of the mutant or covalent dimer and a combination thereof (e.g., the activity to inhibit cell growth) and depending on a subject being treated and a disease condition, the weight and age of the subject, the severity of a disease condition, mode of administration, and the like. The dosage of administration may be within the range of: for example, about 0.1 mg/kg to about 50 mg/kg, about 0.5 mg/kg to about 50 mg/kg, about 1 mg/kg to about 50 mg/kg, about 2 mg/kg to about 50 mg/kg, about 3 mg/kg to about 50 mg/kg, about 5 mg/kg to about 50 mg/kg, about 8 mg/ kg to about 50 mg/kg, about 10 mg/kg to about 50 mg/kg, about 15 mg/kg to about 50 mg/kg, about 20 mg/kg to about 50 mg/kg, about 25 mg/kg to about 50 mg/kg, about 30 mg/kg to about 50 mg/kg, and about 40 mg/kg to about 50 mg/kg of the mutant or covalent dimer.

As used herein, "subject" refers to any animal, including rodents such as mice or rats, primates such as Macaca fascicularis, Macaca mulatta, or Homo sapiens. Preferably, the subject is a primate, more preferably humans. As used herein, "prevention" refers to a method aimed at preventing the onset of a disease or delaying its onset.

As used herein, examples of cancers include, but are not limited to, carcinoma, lymphoma, blastoma, sarcoma, and leukemia. More specific examples include: squamous epithelial cell cancer, small cell lung cancer, non-small cell lung cancer, gastric cancer, pancreatic cancer, glial cell tumors such as glioblastoma and neurofibromatosis, cervical cancer, ovarian cancer, liver cancer, bladder cancer, hepatoma, breast cancer, colon cancer, melanoma, colorectal cancer, endometrial cancer, salivary gland cancer, kidney cancer, renal cancer, prostate cancer, vulvar cancer, thyroid cancer, liver cancer, and various types of head and neck cancers. In a specific embodiment, cancers treated or diagnosed using the method disclosed herein are selected from melanoma, breast cancer, ovarian cancer, renal cancer, gastrointestinal/colon cancer, lung cancer, and prostate cancer. According to a preferred embodiment of the present disclosure, the cancers include, but are not limited to, melanoma, lung cancer, head and neck cancers, colorectal cancer, pancreatic cancer, gastric cancer, renal cancer, bladder cancer, prostate cancer, breast cancer, ovarian cancer, or liver cancer.

If specific techniques or conditions on which procedures are based are not indicated in the examples, the procedures shall be carried out in accordance with the techniques or conditions described in the literature in the art or in accordance with the product specification. The reagents and instruments used without indicating the manufacturers are all conventional products that can be purchased commercially.

### Example 1: Construction and Transformation of Expression Plasmids of Arginine Deiminase and Its Mutants

Based on wild-type ADI (amino acid sequence as set forth in SEQ ID NO: 1, gene sequence as set forth in SEQ ID NO: 5), the researchers of the present disclosure designed mADI mutants as shown in Table 4, and designed and synthesized different mutant genes according to the codon usage preference data of *E. coli* in combination with codon bias, GC content and other factors.

**Table 4**

| mADI Structure | Mutation Type | Amino Acid Sequence | Gene Sequence |
|---|---|---|---|
| ADI (AWT) | Wild-type | SEQ ID NO: 1 | SEQ ID NO: 5 |
| mADI (A1C) | A46C | SEQ ID NO: 2 | SEQ ID NO: 6 |
| mADI(A2C) | E145C | SEQ ID NO: 3 | SEQ ID NO: 7 |
| mADI (A3C) | G337C | SEQ ID NO: 4 | SEQ ID NO: 8 |

The pET-30a plasmid was used as an expression vector for ADI and mADI. Double enzyme digestion confirmed that both the expression vectors for ADI and mADI were consistent with the theoretical sequences. The expression vectors were transformed into the expression host strain BL21 (DE3) using the CaCl₂ method to obtain recombinant expression strains. The recombinant expression strains were screened on kanamycin-containing plates and subjected to IPTG-induced expression to screen high-expression clones, and original seed bank strains were stored at -80°C.

The gene sequences of ADI and mADI were as follows:

**Table 5: Sequence information**

| |
|---|
| SEQ ID NO: 5 |
| AGCGTGTTCGACAGCAAATTTAACGGTATCCACGTTTACAGCGAAATTGGCGAGCTGGAAA CCGTGCTGGTTCACGAACCGGGTCGTGAGATCGACTATATTACCCCGGCGCGTCTGGATGAG CTGCTGTTCAGCGCGATCCTGGAAAGCCACGACGCGTAAGGAGCACCAGAGCTTTGTGA AGATCATGAAAGATCGTGGCATTAACGTGGTTGAACTGACCGACCTGGTTGCGGAGACCTA CGATCTGGCGAGCAAGGCGGCGAAAGAGGAATTCATCGAAACCTTTCTGGAGGAAACCGT GCCGGTTCTGACCGAGGCGAACAAGAAAGCGGTGCGTGCGTTCCTGCTGAGCAAACCGAC CCACGAAATGGTTGAGTTTATGATGAGCGGTATCACCAAGTACGAACTGGGCGTGGAGAGC GAAAACGAGCTGATTGTTGACCCGATGCCGAACCTGTATTTCACCCGTGATCCGTTTGCGAG CGTGGGTAACGGCGTTACCATCCACTTCATGCGTTACATTGTGCGTCGTCGTGAAACCCTGTT TGCGCGTTTCGTGTTTCGTAACCACCCGAAGCTGGTTAAAACCCCGTGGTACTATGACCCGG CGATGAAAATGCCGATCGAGGGTGGCGATGTGTTCATTTATAACAACGAAACCCTGGTGGTT GGTGTTAGCGAGCGTACCGACCTGGATACCATCACCCTGCTGGCGAAGAACATTAAAGCGA ACAAGGAAGTTGAGTTCAAACGTATCGTGGCGATTAACGTTCCGAAGTGGACCAACCTGAT GCACCTGGACACCTGGCTGACCATGCTGGATAAGAACAAATTTCTGTACAGCCCGATCGCGA ACGACGTGTTCAAATTTTGGGACTATGATCTGGTTAACGGTGGCGCGGAACCGCAGCCGCA ACTGAACGGTCTGCCGCTGGATAAACTGCTGGCGAGCATCATTAACAAGGAACCGGTGCTG ATCCCGATTGGTGGCGCGGGTGCGACCGAAATGGAGATCGCGCGTGAGACCAACTTCGACG GCACCAACTACCTGGCGATCAAGCCGGGTCTGGTGATTGGCTATGATCGTAACGAGAAAAC CAACGCGGCGCTGAAGGCGGCGGGTATTACCGTTCTGCCGTTTCACGGCAACCAACTGAGC CTGGGTATGGGCAACGCGCGTTGCATGAGCATGCCGCTGAGCCGTAAAGATGTTAAGTGG (SEQ ID NO: 5) |
| SEQ ID NO: 6 |
| AGCGTGTTCGACAGCAAATTAACGGTATCCACGTTTACAGCGAAATTGGCGAGCTGGAAA CCGTGCTGGTTCACGAACCGGGTCGTGAGATCGACTATATTACCCCGGCGCGTCTGGATGAG CTGCTGTTCAGCTGCATCCTGGAAAGCCACGACGCGTAAGGAGCACCAGAGCTTTGTGA AGATCATGAAAGATCGTGGCATTAACGTGGTTGAACTGACCGACCTGGTTGCGGAGACCTA CGATCTGGCGAGCAAGGCGGCGAAAGAGGAATTCATCGAAACCTTTCTGGAGGAAACCGT GCCGGTTCTGACCGAGGCGAACAAGAAAGCGGTGCGTGCGTTCCTGCTGAGCAAACCGAC CCACGAAATGGTTGAGTTTATGATGAGCGGTATCACCAAGTACGAACTGGGCGTGGAGAGC GAAAACGAGCTGATTGTTGACCCGATGCCGAACCTGTATTTCACCCGTGATCCGTTTGCGAG CGTGGGTAACGGCGTTACCATCCACTTCATGCGTTACATTGTGCGTCGTCGTGAAACCCTGTT TGCGCGTTTCGTGTTTCGTAACCACCCGAAGCTGGTTAAAACCCCGTGGTACTATGACCCGG CGATGAAAATGCCGATCGAGGGTGGCGATGTGTTCATTTATAACAACGAAACCCTGGTGGTT GGTGTTAGCGAGCGTACCGACCTGGATACCATCACCCTGCTGGCGAAGAACATTAAAGCGA ACAAGGAAGTTGAGTTCAAACGTATCGTGGCGATTAACGTTCCGAAGTGGACCAACCTGAT GCACCTGGACACCTGGCTGACCATGCTGGATAAGAACAAATTTCTGTACAGCCCGATCGCGA ACGACGTGTTCAAATTTTGGGACTATGATCTGGTTAACGGTGGCGCGGAACCGCAGCCGCA ACTGAACGGTCTGCCGCTGGATAAACTGCTGGCGAGCATCATTAACAAGGAACCGGTGCTG ATCCCGATTGGTGGCGCGGGTGCGACCGAAATGGAGATCGCGCGTGAGACCAACTTCGACG GCACCAACTACCTGGCGATCAAGCCGGGTCTGGTGATTGGCTATGATCGTAACGAGAAAAC CAACGCGGCGCTGAAGGCGGCGGGTATTACCGTTCTGCCGTTTCACGGCAACCAACTGAGC CTGGGTATGGGCAACGCGCGTTGCATGAGCATGCCGCTGAGCCGTAAAGATGTTAAGTGG (SEQ ID NO:6) |
| SEQ ID NO: 7 |
| AGCGTGTTCGACAGCAAATTTAACGGTATCCACGTTTACAGCGAAATTGGCGAGCTGGAAA CCGTGCTGGTTCACGAACCGGGTCGTGAGATCGACTATATTACCCCGGCGCGTCTGGATGAG CTGCTGTTCAGCGCGATCCTGGAAAGCCACGACGCGTAAGGAGCACCAGAGCTTTGTGA AGATCATGAAAGATCGTGGCATTAACGTGGTTGAACTGACCGACCTGGTTGCGGAGACCTA CGATCTGGCGAGCAAGGCGGCGAAAGAGGAATTCATCGAAACCTTTCTGGAGGAAACCGT GCCGGTTCTGACCGAGGCGAACAAGAAAGCGGTGCGTGCGTTCCTGCTGAGCAAACCGAC CCACGAAATGGTTGAGTTTATGATGAGCGGTATCACCAAGTACGAACTGGGCGTGGAGAGC GAAAACTGCCTGATTGTTGACCCGATGCCGAACCTGTATTTCACCCGTGATCCGTTTGCGAG CGTGGGTAACGGCGTTACCATCCACTTCATGCGTTACATTGTGCGTCGTCGTGAAACCCTGTT TGCGCGTTTCGTGTTTCGTAACCACCCGAAGCTGGTTAAAACCCCGTGGTACTATGACCCGG CGATGAAAATGCCGATCGAGGGTGGCGATGTGTTCATTTATAACAACGAAACCCTGGTGGTT GGTGTTAGCGAGCGTACCGACCTGGATACCATCACCCTGCTGGCGAAGAACATTAAAGCGA ACAAGGAAGTTGAGTTCAAACGTATCGTGGCGATTAACGTTCCGAAGTGGACCAACCTGAT GCACCTGGACACCTGGCTGACCATGCTGGATAAGAACAAATTCTGTACAGCCCGATCGCGA ACGACGTGTTCAAATTTTGGGACTATGATCTGGTTAACGGTGGCGCGGAACCGCAGCCGCA ACTGAACGGTCTGCCGCTGGATAAACTGCTGGCGAGCATCATTAACAAGGAACCGGTGCTG ATCCCGATTGGTGGCGCGGGTGCGACCGAAATGGAGATCGCGCGTGAGACCAACTTCGACG GCACCAACTACCTGGCGATCAAGCCGGGTCTGGTGATTGGCTATGATCGTAACGAGAAAAC CAACGCGGCGCTGAAGGCGGCGGGTATTACCGTTCTGCCGTTTCACGGCAACCAACTGAGC CTGGGTATGGGCAACGCGCGTTGCATGAGCATGCCGCTGAGCCGTAAAGATGTTAAGTGG (SEQ ID NO: 7) |
| SEQ ID NO: 8 |
| AGCGTGTTCGACAGCAAATTAACGGTATCCACGTTTACAGCGAAATTGGCGAGCTGGAAA CCGTGCTGGTTCACGAACCGGGTCGTGAGATCGACTATATTACCCCGGCGCGTCTGGATGAG CTGCTGTTCAGCGCGATCCTGGAAAGCCACGACGCGTAAGGAGCACCAGAGCTTTGTGA AGATCATGAAAGATCGTGGCATTAACGTGGTTGAACTGACCGACCTGGTTGCGGAGACCTA CGATCTGGCGAGCAAGGCGGCGAAAGAGGAATTCATCGAAACCTTTCTGGAGGAAACCGT GCCGGTTCTGACCGAGGCGAACAAGAAAGCGGTGCGTGCGTTCCTGCTGAGCAAACCGAC CCACGAAATGGTTGAGTTTATGATGAGCGGTATCACCAAGTACGAACTGGGCGTGGAGAGC GAAAACGAGCTGATTGTTGACCCGATGCCGAACCTGTATTTCACCCGTGATCCGTTTGCGAG CGTGGGTAACGGCGTTACCATCCACTTCATGCGTTACATTGTGCGTCGTCGTGAAACCCTGTT TGCGCGTTTCGTGTTTCGTAACCACCCGAAGCTGGTTAAAACCCCGTGGTACTATGACCCGG CGATGAAAATGCCGATCGAGGGTGGCGATGTGTTCATTTATAACAACGAAACCCTGGTGGTT GGTGTTAGCGAGCGTACCGACCTGGATACCATCACCCTGCTGGCGAAGAACATTAAAGCGA ACAAGGAAGTTGAGTTCAAACGTATCGTGGCGATTAACGTTCCGAAGTGGACCAACCTGAT GCACCTGGACACCTGGCTGACCATGCTGGATAAGAACAAATTTCTGTACAGCCCGATCGCGA ACGACGTGTTCAAATTTTGGGACTATGATCTGGTTAACGGTGGCGCGGAACCGCAGCCGCA ACTGAACGGTCTGCCGCTGGATAAACTGCTGGCGAGCATCATTAACAAGGAACCGGTGCTG ATCCCGATTGGTGGCGCGGGTGCGACCTGCATGGAGATCGCGCGTGAGACCAACTTCGACG GCACCAACTACCTGGCGATCAAGCCGGGTCTGGTGATTGGCTATGATCGTAACGAGAAAAC CAACGCGGCGCTGAAGGCGGCGGGTATTACCGTTCTGCCGTTTCACGGCAACCAACTGAGC CTGGGTATGGGCAACGCGCGTTGCATGAGCATGCCGCTGAGCCGTAAAGATGTTAAGTGG |
| (SEQ ID NO: 8) |

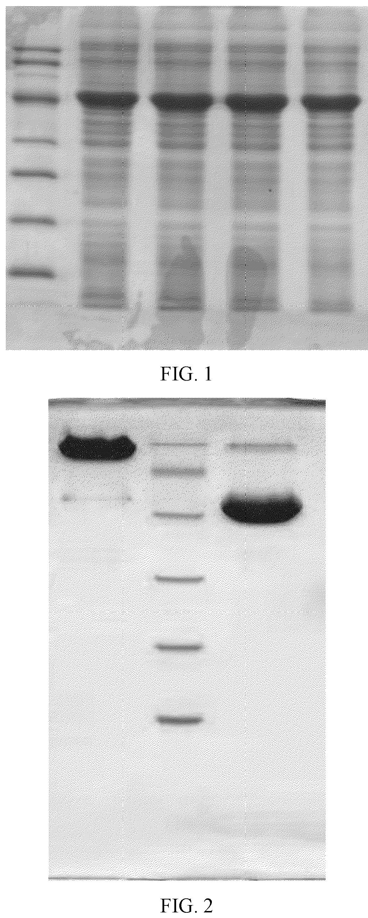

Researches showed that, as shown in FIG. 1, through shake cultivation of the constructed strains in a triangular flask and IPTG-induced expression, wild-type ADI (AWT) and mutant mADI strains were successfully constructed. Target protein bands corresponding to ADI or mADI subunits were observed at approximately 46 kDa with similar expression levels across samples.

### Example 2: Preparation and Purification of Arginine Deiminase and Its Mutants

Inclusion bodies containing ADI or mADI were obtained by fermentation. Specifically, original strains of ADI or mADI were subjected to two-step scale-up fermentation in a fermenter. The conditions were controlled at 30-34°C and pH 7.0-7.4 until the OD₆₀₀ value exceeded 30, followed by heating to 36-38°C and added IPTG to 0.5 mmol/L. Induction was continued for more than 3 hours to allow accumulation of ADI or mADI. Cells were harvested by centrifugation and then stored below -15°C.

The ADI or mADI inclusions produced by the above fermentation were denaturated and refolded to obtain active ADI or mADI crude products. Specifically, the fermented bacteria were suspended in a bacterial disruption buffer containing 20 mmol/L K₂HPO₄ and 5 mmol/L EDTA, and crushed by high-pressure homogenization at 500-800 bar; and ADI or mADI inclusion bodies were collected by centrifugation. The inclusion body pellets were washed with a bacterial disruption buffer containing 1-3% Triton X-100 followed by a buffer containing 20 mmol/L K₂HPO₄. The washed inclusion bodies were dissolved in a denaturation buffer containing 8 mol/L urea, 20 mmol/L K₂HPO₄ and 5 mM DTT, and stirred and dissolved at room temperature for more than 1 h. The supernatant was collected by centrifugation and subjected to dilution refolding by slowly diluting at a ratio of 1:30 to 1:50 (V/V) into a refolding buffer (20 mmol/L K₂HPO₄, pH 7.0-7.5), and stirred at 10-15°C for more than 36 hours to obtain active ADI or mADI.

Multi-step chromatography was used for further purification to obtain high-purity ADI or mADI. In short, initial purification was performed by anion exchange chromatography, followed by hydrophobic chromatography or anion exchange chromatography under various salt concentrations for elution or flow-through for further purification. Finally, ultrafiltration was used for buffer exchange and concentration to obtain purified ADI or mADI samples at the desired concentration.

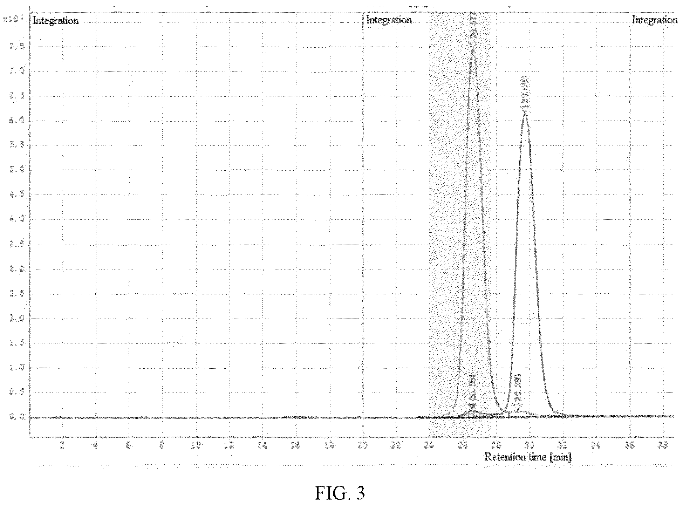

The results of preparation and purification studies, as shown in FIG. 2, indicated that the purity of ADI or mADI exceeded 95% as determined by non-reducing SDS-PAGE and SEC-HPLC. The representative mADI structure A1C showed a single band at approximately 92 kDa, indicating that the target protein A1C existed in the form of a covalent dimer. In contrast, wild-type ADI (AWT), which did not form a covalent dimer, appeared as a single band at approximately 46 kDa under denaturing conditions, indicating a monomeric form. Further studies using SEC-HPLC, as shown in FIG. 3, showed that when the mADI structure A1C existed as the form of a monomeric subunit after DTT reduction treatment, the retention time in SEC-HPLC was prolonged, which was consistent with the retention time of the wild-type ADI structure AWT, further confirming that A1C existed in the form of a covalent dimer and was more stable in different buffers than the non-covalent dimer of AWT.

### Example 3: Enzyme Activity Assay of Samples

In Example 3, the enzyme activity was determined by a modified method. The method includes the following steps.

An appropriate amount of ADI or mADI enzyme was added to a solution containing 10 mol/L L-Arg and 0.1 mol/L sodium phosphate buffer (pH 6.0), mixed well at 37°C for 5 min. A mixed acidic iron-containing solution was added to terminate the reaction. The reaction was incubated in a 100°C metal bath for 10 min. The absorbance value was measured at a wavelength of 530 nm. The citrulline content in the reaction was determined using a standard curve prepared with known concentrations of citrulline.

Enzyme activity unit (U) was defined as the amount of enzyme required to produce 1 µmol of L-citrulline within 1 min at 37°C, i.e., one unit (U).

The results of the ADI or mADI enzyme were shown in Table 6.

**Table 6**

| Sample Name | Enzyme Activity (U/mg) |
|---|---|
| ADI (AWT) | 30.2 |
| mADI (A1C) | 29.6 |
| mADI (A2C) | 29.4 |
| mADI (A3C) | 26.3 |

The research results of the ADI or mADI enzyme showed that, after mutation, mADI forms a covalent dimer. Except for mADI with the A3C structure, whose activity was slightly lower than wild-type ADI, the enzyme activities of mADI (A1C and A2C) were essentially comparable to wild-type ADI (AWT).

### Example 4: Preparation of Conjugates of Arginine Deiminase and Its Mutants

In Example 4, the conjugates of arginine deiminase and its mutants were prepared using monomethoxy polyethylene glycol (m-SPA) with molecular weights of 2 kDa, 5 kDa, 10 kDa, and 20 kDa, having an active group of succinimidyl propionate (SPA) as the active group. The resulting conjugated mixtures were further separated and purified by ultrafiltration and ion exchange chromatography to obtain a purified ADI or mADI PEG-modified conjugate.

The purified ADI or mADI protein was diluted with 100 mol/L phosphate modification buffer with pH of 7.5-9.5 till the concentration of the ADI or mADI protein was in the range of 5 to 10 mg/ml. According to different molar ratios (1: 20 to 1: 160) of the ADI or mADI protein to polyethylene glycol M-SPA-5K, polyethylene glycol was weighed, and mixed and stirred at room temperature for more than 1 h to obtain mixtures of modified conjugates with different degree of modification.

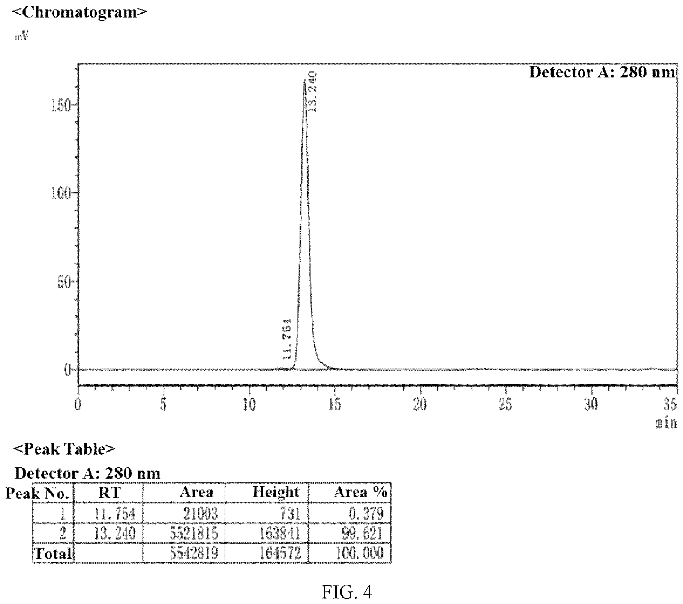

The resulting mixtures of modified conjugates with different degree of modification were first subjected to ultrafiltration using 30 kDa or 100 kDa hollow fiber columns to remove unbound PEG and by-products. Ion exchange chromatography was then employed to remove unmodified ADI or mADI and endotoxins. Finally, 30 kDa hollow fiber columns were used to replace the buffer with 20 mmol/L phosphate buffer (pH 7.0-8.0), which was aliquoted to obtain PEG-modified conjugates of ADI or mADI (e.g., PEG-ADI-5K, PEG-mADI-5K, PEG-mADI-10K, PEG-mADI-20K), as shown in FIG. 4. The results of purity detection by SEC-HPLC showed that the purified PEG-AlC-5K had the purity up to 99%.

### Example 5: Quality Evaluation of Conjugates of Arginine Deiminase and Its Mutants

According to the method provided in Example 3, the enzyme activity and content of samples before and after modification were determined.

The average degree of PEGylation was determined based on the principle described by Kantian et al. (On-line characterization of polyethylene glycol-modified proteins). Briefly, the absorption values of the protein in refractive index (RI) and UV were linearly related to the protein concentration, and the RI absorption value of PEG was also linearly related to its concentration. The PEG moiety and protein moiety after PEGylation do not interfere with each other's absorbance in RI or UV. Therefore, the average degree of modification was calculated using SEC-HPLC coupled with RI and UV detectors.

The results of average degree of modification and enzyme activity detection were shown in Table 7. For PEG-A1C-5K or PEG-AWT-5K samples with different degree of modification obtained by modifying at different feed ratios, with the increase in the average degree of modification, the specific enzyme activity of PEG-mADI-5K and PEG-ADI-5K showed a decreasing trend compared to the unmodified A1C and AWT. However, within the same degree of modification range, the enzyme activity retention of PEG-A1C-5K was significantly higher than that of PEG-AWT-5K. This may be attributed to the fact that when the average degree of modification of PEG-AWT-5K was higher than 10 or more (e.g., 11.5), the structure of PEG-AWT-5K became unstable and began to depolymerize, resulting in a significant decrease in enzyme activity. In contrast, when the average degree of modification of PEG-AlC-5K was higher than 10 or more, the A1C structure still did not undergo depolymerization, and the enzyme activity retention was relatively high. The decrease in enzyme activity might be due to an increase in the modification degree, which affected the binding of the enzyme to the substrate or caused inactivation of some enzymatically active regions after being modified.

The above quality evaluation further proved that the structure of the covalent dimeric arginine deiminase mutant was more stable and its enzyme activity more durable than that of the non-covalent dimeric arginine deiminase.

**Table 7**

| Structure | Average Degree of Modification | Activity (U/mg) | Enzyme Activity Retention (%) |
|---|---|---|---|
| PEG-A1C-5K | 0 | 29.6 | 100 |
| | 7.0 | 29.2 | 98.6 |
| | 9.2 | 28.3 | 95.6 |
| | 10.6 | 22.9 | 77.4 |
| | 11.1 | 21.4 | 72.3 |
| | 12.4 | 20.1 | 67.9 |
| | 14.4 | 19.6 | 66.2 |
| | 15.2 | 18.3 | 61.8 |
| | 16.6 | 15.7 | 53.0 |
| PEG-AWT-5K | 0 | 30.2 | 100 |
| | 7.2 | 25.3 | 83.77 |
| | 9.7 | 22.2 | 73.51 |
| | 10.8 | 17.1 | 56.62 |
| | 11.5 | 11.4 | 37.75 |
| | 14 | 10.04 | 33.25 |
| | 16.1 | 6.36 | 21.06 |
| | 17.1 | 4.77 | 15.79 |

The binding affinity of the modified samples to A1C rabbit polyclonal antibodies was detected by a competitive ELISA method. Briefly, plates were coated with A1C rabbit polyclonal antibodies (10 µg/ml, 100 µl/well), washed with PBS three times, blocked with 2% BSA overnight, and washed with PBS three times. Biotin-A1C was diluted with PBS to 100 ng/ml. The samples A1C, PEG-A1C-2K, PEG-A1C-5K, PEG-A1C-10K, and PEG-A1C-20K were diluted to 100 ng/ml, and then mixed with Biotin-A1C in equal volumes. The mixed sample was added at 50 µl/well, respectively, and shaken at 25°C for 30 min. With PBS as a control, the plates were washed with PBS five times, and Avidin-HRP (1: 2500 dilution) was added at 100 µl/well and shaken at 150 rpm, 25°C for 30 min. A TMB developing solution A was added at 50 µl/well followed by a TMB developing solution B at 50 µl/well, and incubated for 10 min at room temperature in the dark. A terminating solution was added at 50 µl/well to terminate the reaction. OD values at 450 nm were read within 30 min using a microplate reader.

The results were shown in Table 8. After A1C was PEGylated, PEG-modified products of different sizes significantly reduced the immunogenicity (the higher the OD value, the closer it is to the OD value of PBS, and the lower the immunogenicity). A1C modified with 5K, i.e., PEG-A1C-5K, had the best effect on the reduction of the immunogenicity.

**Table 8**

| PBS | PEG-A1C-2K | PEG-A1C-5K | PEG-A1C-10K | PEG-A1C-20K | A1C |
|---|---|---|---|---|---|
| 1.922 | 1.691 | 1.814 | 1.799 | 1.564 | 0.456 |

### Example 6: In Vitro Proliferation Inhibition Assay on Different Tumor Cells

Tumor cell lines in the logarithmic growth phase, such as murine melanoma B16F10 cells and human liver cancer SK-Hep-1 cells, were harvested, diluted in complete culture medium, and seeded in 96-well plates. The PEG-A1C-5K sample was serially diluted, added at 10 µL in equal volume per well, and continued for 3 days of culture. the cell proliferation inhibition at various concentrations was assessed using the CCK-8 method, and the IC₅₀ values were calculated.

**Table 9**

| Cell Type | Cell Line | Imax (Maximum Inhibition Rate) | IC₅₀ (MG/Ml) |
|---|---|---|---|
| Murine melanoma cells | B16F10 | 65.92% | 0.07±0.01 |
| Human liver cancer cells | SK-Hep-1 | 98.75% | 0.17±0.01 |
| Leukemia cells | KG-1 | 85.21% | 0.26±0.05 |
| Pancreatic cancer cells | PANC-1 | 78.65% | 0.005±0.0016 |
| Colorectal cancer cells | HCT-116 | 71.23% | 0.025±0.016 |
| Small cell lung cancer cells | NCI-H446 | 86.47% | 0.06±0.02 |

As shown in Table 9, Imax represents the maximum growth inhibition rate, with higher values indicating better efficacy. The results demonstrated that PEG-A1C-5K, PEG-A2C-5K, PEG-A3C-5K, and PEG-AWT-5K significantly inhibited the proliferation of all tested cell lines.

### Example 7: Single-Dose Pharmacokinetic Evaluation of Conjugates of Arginine Deiminase and Its Mutants in Rats

Pharmacokinetics (PK) of PEG-AWT-5K and PEG-A1C-5K were evaluated in rats, with 6 rats in each group. PEG-AWT-5K and PEG-AlC-5K were injected into the tail vein of each rat in each group at a dosage of 1 mg/kg body weight, respectively. Blood samples were collected on days 1, 3, 7, 10, and 14 after dosing for PK analysis.

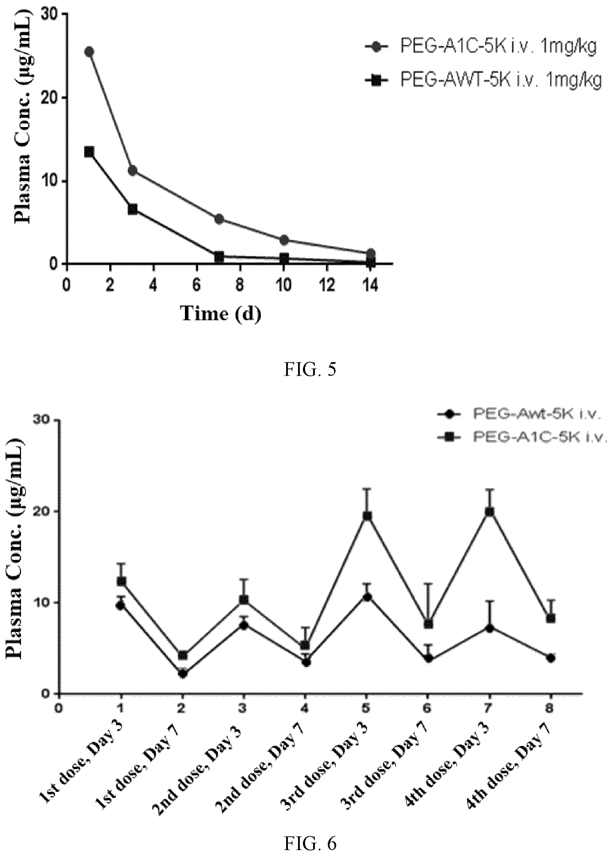

The results were shown in FIG. 5. The PEG-A1C-5K group maintained for more than 10 days, while the PEG-AWT-5K group maintained for 7 days. It suggested that PEG-AlC-5K exhibited a significantly longer blood retention time than PEG-AWT-5K.

### Example 8: Multiple-Dose Pharmacokinetics and Immunogenicity Evaluation of Conjugates of Arginine Deiminase and Its Mutants in Rats

12 male SD rats were randomly divided into 2 groups. Each group received weekly intravenous injections of PEG-AWT-5K or PEG-A1C-5K at 1.0 mg/kg for four weeks. Blood samples were collected on days 3 and 7 after each dose to assess PK and anti-drug antibody (ADA) levels.

The results were shown in FIG. 6. After the third dosing, PEG-A1C-5K exhibited drug accumulation in plasma, while no accumulation was observed for PEG-AWT-5K. This suggested that PEG-A1C-5K had longer and more stable blood retention than PEG-AWT-5K. In addition, it was found from antibody detection that no anti-protein antibodies were detected in the PEG-A1C-5K group, while approximately 30% of PEG-AWT-5K produced anti-protein antibodies, indicating that after PEG modification, the covalent dimeric arginine deiminase formed after mutation, the A1C structure, exhibited superior epitope shielding compared to the non-mutated AWT structure.

### Example 9: Single-Dose Pharmacokinetic Evaluation of Conjugates of Arginine Deiminase and Its Mutants in Rhesus Monkeys

The PK of PEG-AlC-5K was evaluated in rhesus monkeys, with two animals in each group. Each animal in each group was injected with PEG-AlC-5K intravenously at the doses of 1 mg/kg and 3 mg/kg, respectively. Plasma was collected before dosing and 5 min (±1 min), 1 h, 2 h, 4 h, 8 h, 24 h, 48 h, 72 h, 168 h, 240 h, 336 h, 408 h and 504 h after dosing, to assess the plasma concentration and PK.

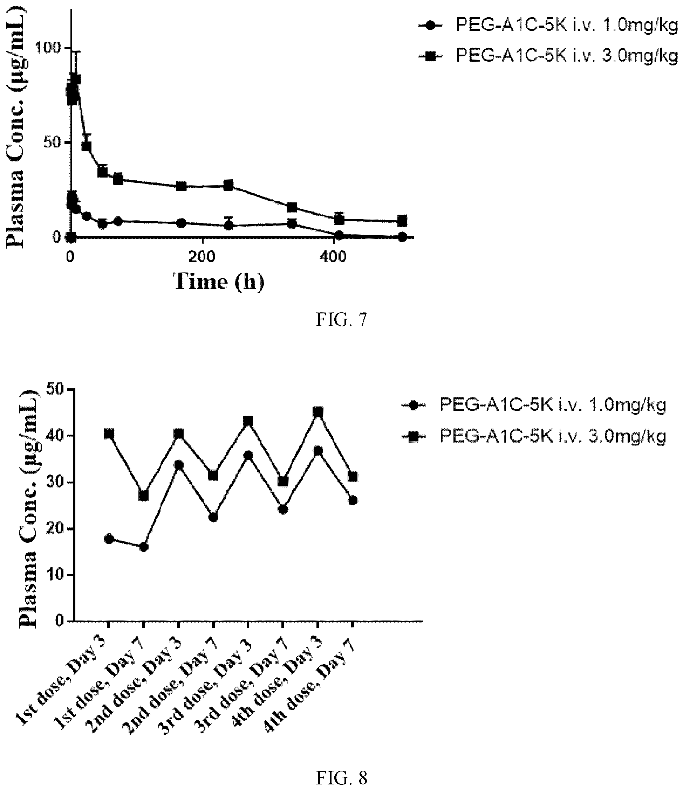

The results were shown in FIG. 7. PEG-AlC-5K maintained for more than 21 days, with a half-life of 131-192 h.

### Example 10: Multiple-Dose Pharmacokinetics and Immunogenicity Evaluation of Conjugates of Arginine Deiminase and Its Mutants in Rhesus Monkeys

PK and immunogenicity of PEG-A1C-5K were evaluated in rhesus monkeys, with 2 animals in each group. Each animal in each group was administrated intravenously with PEG-AlC-5K at the doses of 1 mg/kg and 3 mg/kg, respectively. Blood samples were collected 3 days and 7 days after dosing for PK and ADA detection and analysis.

The results were shown in FIG. 8. The plasma concentration of PEG-A1C-5K in rhesus monkeys increased with the dosage. In addition, it was found from the antibody detection that only low-titer (1:10) anti-protein antibodies were present in the PEG-A1C-5K group.

### Example 11: In Vivo Inhibition Effect of Conjugates of Arginine Deiminase Mutants on Tumor Cells

After subcutaneous inoculation of murine melanoma B16F10 cells in female C57 mice, they were randomly divided into 5 groups. The dosing regimen was shown in Table 10. The tumor volume was measured 2-3 times a week. At study end, blood was collected, animals were euthanized, tumors were excised and weighed, and serum arginine and citrulline levels were analyzed.

**Table 10**

| **Groups** | **Number of Animals** | **Treatment** | **Dose (mg/kg)** | **Dosing Route** | **Dosing Frequency** | **Dosing Cycle (Days)** |
|---|---|---|---|---|---|---|
| 1 | 6 | Model control group | / | Intravenous injection | Once a week | 20 |
| 2 | 6 | PEG-AWT-5K | 1 | Intravenous injection | Once a week | 20 |
| 3 | 6 | PEG-A1C-5K | 1 | Intravenous injection | Once a week | 20 |
| 4 | 6 | PEG-A2C-5K | 1 | Intravenous injection | Once a week | 20 |
| 5 | 6 | PEG-A3C-5K | 1 | Intravenous injection | Once a week | 20 |

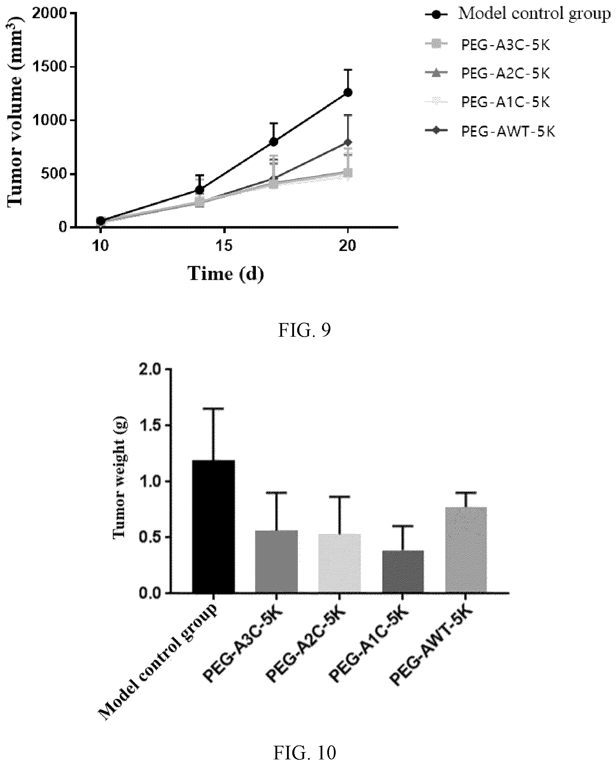

The results of tumor volume and tumor weight were shown in FIG. 9 and FIG. 10. Compared with the model control group, PEG-AWT-5K, PEG-A1C-5K, PEG-A2C-5K, and PEG-A3C-5K all inhibited the tumor growth. Notably, the inhibitory effects of PEG-A1C-5K, PEG-A2C-5K, and PEG-A3C-5K on tumor growth were significantly better than that of PEG-AWT-5K.

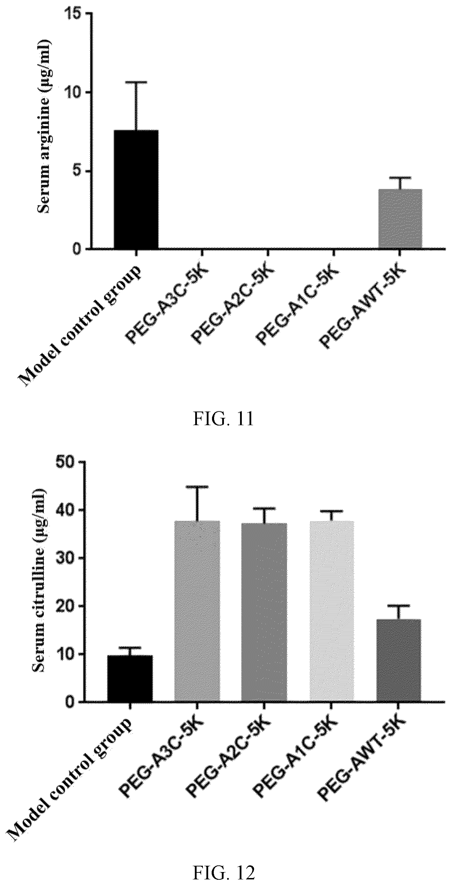

The results of serum arginine and citrulline levels were shown in FIG. 11 and FIG. 12. Compared with the model control group, PEG-AWT-5K, PEG-A1C-5K, PEG-A2C-5K, and PEG-A3C-5K all reduced arginine in the serum and maintained citrulline products in the serum. Notably, PEG-A1C-5K, PEG-A2C-5K, PEG-A3C-5K maintained low levels of arginine and high levels of citrulline, which were significantly better than those of PEG-AWT-5K. This might be related to the production of anti-protein antibodies in PEG-AWT-5K after the third dosing, resulting in reduced efficacy, consistent with its weaker tumor inhibition.

In the description of the present specification, the description of referring terms such as "an example", "some examples", "an embodiment", "a specific embodiment" and "some embodiments" integrate particular features, structures, materials or characteristics described in combination of the embodiments or examples and included in at least one embodiment or example of the present disclosure. In the present specification, the schematic description of the above terms does not necessarily refer to the same embodiment or example. Furthermore, the described particular features, structures, materials or characteristics can be integrated with any one or more embodiments or examples in a proper manner. In addition, in the absence of contradiction, a person skilled in the art can integrate and combine different embodiments or examples described in this specification and the features of different embodiments or examples.

Although the embodiments of the present disclosure have been shown and described above, it can be understood that the above embodiments are exemplary and should not be construed as limiting the present disclosure. A person of ordinary skill in the art can make changes, modifications, substitutions and variations to the above-mentioned examples within the scope of the present disclosure.

## Claims

1. An arginine deiminase mutant, comprising an inter-subunit covalent disulfide bond formed between arginine deiminase subunits via mutation at an amino acid site on arginine deiminase.

2. The arginine deiminase mutant according to claim 1, wherein the mutant exists in the form of a covalent dimer, the covalent dimer formed between non-covalent homodimeric arginine deiminase subunits by site-directed mutagenesis at an amino acid site on arginine deiminase.

3. The arginine deiminase mutant according to claim 1, wherein, based on a sequence derived from *Mycoplasma hominis* or a homologous sequence thereof, at least one amino acid at positions 46, 145 and 337 of a sequence as set forth in SEQ ID NO: 1 is mutated to cysteine in the mutant.

4. The arginine deiminase mutant according to claim 3, wherein the sequence derived from *Mycoplasma hominis* or the homologous sequence thereof is the sequence as set forth in SEQ ID NO: 1, or a sequence having at least 80%, 81%, 82%, 83%, 84%, 85%, 86%, 87%, 88%, 89%, 90%, 91%, 92%, 93%, 94%, 95%, 96%, 97%, 98%, or 99% homology to the sequence as set forth in SEQ ID NO: 1.

5. The arginine deiminase mutant according to claim 1, wherein the mutant is derived from *Mycoplasma hominis,* an amino acid at position 46 of a sequence as set forth in SEQ ID NO: 1 is mutated to cysteine, and an amino acid sequence of the arginine deiminase subunit of the mutant is as set forth in SEQ ID NO: 2.

6. The arginine deiminase mutant according to claim 1, wherein the mutant is derived from *Mycoplasma hominis,* an amino acid at position 145 of a sequence as set forth in SEQ ID NO: 1 is mutated to cysteine, and an amino acid sequence of the arginine deiminase subunit of the mutant is as set forth in SEQ ID NO: 3.

7. The arginine deiminase mutant according to claim 1, wherein the mutant is derived from *Mycoplasma hominis,* an amino acid at position 337 of a sequence as set forth in SEQ ID NO: 1 is mutated to cysteine, and an amino acid sequence of the arginine deiminase subunit of the mutant is as set forth in SEQ ID NO: 4.

8. A covalent dimer, comprising the arginine deiminase mutant according to any one of claims 1 to 7.

9. A covalent conjugate, comprising:
the arginine deiminase mutant according to any one of claims 1 to 7 or the covalent dimer according to claim 8, and
a modifying group chemically coupled thereto.

10. The covalent conjugate according to claim 9, wherein the modifying group forms an amide bond with ε-amino of a lysine residue or α-amino of an N-terminal amino acid residue of the arginine deiminase mutant.

11. The covalent conjugate according to claim 9, wherein the modifying group is selected from at least one of polyethylene glycol, dextran, and a peptide repeat sequence, having an active group.

12. The covalent conjugate according to claim 9, wherein the modifying group has a molecular weight of 2 KDa to 20 KDa, preferably 5 KDa.

13. The covalent conjugate according to claim 9, wherein the number of the modifying group ranges from 9 to 20.

14. The covalent conjugate according to claim 11, wherein the active group is selected from at least one of succinimidyl carbonate, succinimidyl propionate, succinimidyl acetate, and succinimidyl succinate; and preferably, the active group is succinimidyl propionate.

15. The covalent conjugate according to claim 9, wherein the modifying group is polyethylene glycol having an active group.

16. The covalent conjugate according to claim 15, wherein the polyethylene glycol is linear or branched, and the polyethylene glycol is preferably linear.

17. The covalent conjugate according to claim 15, wherein the polyethylene glycol has a molecular weight of 2 KDa to 20 KDa, preferably 5 KDa.

18. The covalent conjugate according to claim 15, wherein the active group is selected from at least one of succinimidyl carbonate, succinimidyl propionate, succinimidyl acetate, and succinimidyl succinate.

19. The covalent conjugate according to claim 15, wherein the active group is succinimidyl propionate.

20. The covalent conjugate according to any one of claims 9 to 19, exhibiting at least one of the following properties:
(a) the conjugate has an enzyme activity retention rate of not less than 50% that of the arginine deiminase mutant in the form of a covalent dimer;
(b) the conjugate has low immunogenicity and prolonged in vivo half-life; and
(c) the conjugate is capable of reducing an in vivo arginine level.

21. A pharmaceutical composition, comprising:
the arginine deiminase mutant according to any one of claims 1 to 7, the covalent dimer according to claim 8, or the covalent conjugate according to any one of claims 9 to 19; and
a pharmaceutically acceptable carrier.

22. Use of the arginine deiminase mutant according to any one of claims 1 to 7, the covalent dimer according to claim 8, the covalent conjugate according to any one of claims 9 to 20, or the pharmaceutical composition according to claim 21 in the manufacture of a medicament for the treatment of a tumor.

23. The use according to claim 22, wherein the tumor is an arginine-dependent tumor.

24. The use according to claim 22, wherein the tumor is ASS-deficient, P53 wild-type, or a combination thereof.

25. The use according to claim 22, wherein the tumor is selected from at least one of hepatocellular carcinoma, melanoma, pancreatic cancer, colorectal cancer, acute myeloid leukemia, and small cell lung cancer.

26. Use of the arginine deiminase mutant according to any one of claims 1 to 7, the covalent dimer according to claim 8, the covalent conjugate according to any one of claims 9 to 20, or the pharmaceutical composition according to claim 21 in the prevention or treatment of a tumor.

27. The use according to claim 26, wherein the tumor is an arginine-dependent tumor.

28. The use according to claim 26, wherein the tumor is ASS-deficient, P53 wild-type, or a combination thereof.

29. The use according to claim 26, wherein the tumor is selected from at least one of hepatocellular carcinoma, melanoma, pancreatic cancer, colorectal cancer, acute myeloid leukemia, and small cell lung cancer.

30. A method for preventing or treating a tumor, comprising administering to a subject the arginine deiminase mutant according to any one of claims 1 to 7, the covalent dimer according to claim 8, the covalent conjugate according to any one of claims 9 to 20, or the pharmaceutical composition according to claim 21.

31. The method according to claim 30, wherein the tumor is an arginine-dependent tumor.

32. The method according to claim 30, wherein the tumor is ASS-deficient, P53 wild-type, or a combination thereof.

33. The method according to claim 30, wherein the tumor is selected from at least one of hepatocellular carcinoma, melanoma, pancreatic cancer, colorectal cancer, acute myeloid leukemia, and small cell lung cancer.
